# EUROPEAN PATENT SPECIFICATION

(11) **EP 1 764 050 B1**
(45) Date of publication and mention of the grant of the patent: **26.05.2010**
(21) Application number: 06120844.3
(22) Date of filing: 18.09.2006
(51) Int. Cl.: A61B 17/72, A61B 17/66, A61B 17/00

(54) **Intramedullary fixation pin**
Intramedullärer Marknagel zur Fixierung
Clou intramédullaire de fixation

(30) Priority: 20.09.2005 TR 200503768
(43) Date of publication of application: 21.03.2007
(73) Proprietor: HAVITCIOGLU, Hasan, Izmir (TR)
(72) Inventor: Havitcioglu, Hasan, Zeytinalani Izmir (TR); Baran, Onder, Zeytinalani Izmir (TR); Uzun, Bora, Karsiyaka Izmir (TR); Oflaz, Hakan, Balcova Izmir (TR)
(74) Representative: Iskender, Ibrahim

(56) References cited:
- WO-A-98/47438
- WO-A-03/043512
- DE-A1- 19 615 103
- DE-A1- 19 810 640
- FR-A1- 2 726 460
- US-A- 5 415 660
- US-A- 5 626 581
- US-B1- 6 730 087

## Description

### Technical Field

The invention relates to bone fixation and extensions.

The invention relates particularly to a bone intramedullary fixation pin that may be used along with the devices used for bone fractures in fracture fixation, extremity extensions, tumor prosthesis extensions and deformity corrections.

### Background of the Invention

Bone intramedullary pins have been developed for use in fracture fixation, extremity extensions, tumor prosthesis extensions and deformity corrections.

With the help of the bone fixation pins, the bones may be fixed internally or externally by an operation. There exist external fixation devices used to fix the bone externally as well as the internal fixation materials.

Disc, external fixer and intramedullary pin are the most commonly used tools for the case involving the development of shortness for various reasons. In the extension carried out with said external fixers, the most frequently encountered complication is the pin path infection. The neurovascular structures might be damaged during both the placement of the screws or wires and the distraction.

It is not possible to use the intramedullary pins alone for extremity extensions. Following the performance of osteotomy from the suitable zone in the bone to be extended, the intramedullary pin is applied as proximally locked and distally non-locked, an external fixer is separately placed and the extension is achieved with the help of this external fixer. However, many studies have been conducted to develop intramedullary pins that could provide extension without the need to use the external fixer, as a result of the presence of the aforesaid complications in the use of the external fixers.
In all these studies, numerous complications have emerged such as the hardships in patient motility, inadequacy of the mechanisms providing the extensions, difficulties in application and infection.

In addition to their use in fracture fixation, intramedullary pins have recently been used also for the preservation of the fracture consolidation following the extensions carried out by the external fixers. It is important with respect to providing a fast consolidation as well as the comfort of the patient.

This fixation pin is used to fix the cortex and the medulla of the bone. Following the placement of these fixation pins intramedullary, lower and upper bone fragments are fixed by means of the pins passing through the cortex and the medulla. The pins passing from the bottom and the top secure the tubular shaft and the intramedullary pin in a way to pass through the external and internal diameter of the bone.

In recent years, new mechanical and externally driven designs have become available for the bone extensions. However, among these, in the mechanical systems, the leg of the patient may encounter uncontrolled mechanical movements and this may lead to undesirable results. Due to its requirement for excessive rotational movement, it has such drawbacks as the difficulty in application. Moreover, as a result of the excessive rotational movement, there exists the risk for the fresh bone clavicles that form between the ends of fracture and the callus tissue to break. It may be necessary for the applications to be made under the supervision of the physician and the hospital.

In the efforts made with the telemetric system, the amount of extension may be limited. There is no control mechanism available to enable the extension procedure to be controlled and to prevent reflex extension as a result of the stimulus.

Furthermore, the WO 03/043512 numbered patent relates to a distraction device for moving a one-piece or two-piece or seperated bone apart in order to extend or bridge a bone gap, optionally in the form of an intramedullary nail which can be inserted into a medullary space of a bone and has at least two elements which can be moved axially in relation to one another, the first element being connected to the second element via at least one detent device for securing a distraction movement.

### Object of the Invention

Based on the known status of the art, the object of the invention is to eliminate the present disadvantages thanks to the improvements provided in the bone intramedullary fixation pin used in the fracture fixation, extremity extensions, tumor prosthesis extension and deformity corrections.

Another object of the invention is to provide ease in the patient motility in extremity extensions.

Still another object of the invention is to minimize the infection risk.

Still another object of the invention is to provide the ability to control the extension procedure externally or to enable the patient to carry out the extension by himself/herself in extremity extensions. In this way, many positive contributions have been provided for the orthopedic surgical practices and the patient health.

In order to achieve the said objectives, innovations have been provided in the bone intramedullary fixation pins used in bone fixations and extensions comprising at least one extension shaft and at least two fixation pins that attach said extension shaft from one end to the bone.

In accordance with the present invention, there is provided a bone intramedullary fixation pin according to independent claim 1.

According to the invention, at least one cascaded upper body has been provided, which has a cavity where said extension shaft can move, has one end connected to the bone by means of the fixation pins and has a hollow form, in order to enable said extension shaft to move depending on the pressure force exerted by the body.

According to the invention, a plurality of pin steps have been realized, which push the movement pins of the hollow end of the extension stopper body inwards, are formed on the inner sides of said cascaded upper body and are placed in the form of projections at reciprocal positions with certain intervals.

According to a preferred embodiment of the invention, at least two extension springs have been formed, which exert pushing force on said extension shaft, are connected to the end of said extension shaft and have the capability of force damping and pressure imparting.

According to a preferred embodiment of the invention, at least two stimulus springs have been formed, which are placed and compressed between said extension stopper body and the cascaded upper body and have the capability of force damping and pressure imparting, in order to enable the extension stopper body to push the extension shaft by compressing the extension springs by virtue of the pressure load imparted by the body on the cascaded upper body pushing the pin steps and the movement pins.

According to a preferred embodiment of the invention, at least one compression step has been formed inside an upper part of said cascaded upper body, in order to enable the control spring to be compressed between said extension stopped body and the cascaded upper body.

According to a preferred embodiment of the invention, at least one load control spring has been formed, which is located between the open end of the said extension stopper body and the cascaded upper body and is capable of shock absorbing, in order to prevent the extension shaft from moving beyond control in case of an unbalanced load imparted on said cascaded upper body or of the springs being excessively compressed.

According to a preferred embodiment of the invention, at least one protective membrane has been formed, which is connected between an open end of said cascaded upper body and the extension shaft, which is resistant to corrosion and capable of isolation, in order to prevent the body fluid from leaking through the gap remaining between said cascaded upper body and the extension shaft.

### Description of the Drawings

Figure-1a is the front sectional view of the intramedullary pin operating on mechanical system according to a representative application of the invention.
Figure-1 b is the disassembled view of the intramedullary pin operating on mechanical system according to a representative application of the invention provided in Figure-1a.

### Reference Numbers

- 1: Cascaded body
- 1.1: Pin steps
- 1.2: Compression step
- 2: Extension shaft
- 2.1: Movement pin
- 2.2: Hollow end
- 3: Extension stopper body
- 3.1: Safety stopper
- 4: Extension spring
- 5: Stimulus spring
- 6: Fixation pins
- 7: Protective membrane
- 8: Load control spring

### Detailed Description of the Invention

The embodiment of the invention illustrated in the figures is a mechanical system telemetric intramedullary pin apparatus that operates without any external intervention. In Figure-1a, upper part of the intramedullary pin with a constant diameter and the lower part of the intramedullary pin that may engage into this body are seen. At the end portion of the extension shaft (2), the extension shaft (2) is enabled to automatically move within certain limits according to the extension in the bone, owing to a hollow end (2.2) which is flexible and has the capability of being pashed inwards.

The extension shaft (2) is provided to gradually move within certain limits, owing to a plurality of the movement pins (2.1), which are formed on the hollow end (2.2), move by being engaged into reciprocal holes and are in the form of projections at reciprocal positions placed at determined intervals. The movement pins (2.1) are designed in a unidirectional manner.

The extension shaft (2) is provided to move, owing to preferably two extension (distraction) springs (4), which impart pushing force on the extension shaft (2), are connected to the end of said extension shaft (2) and are capable of force damping and pressure imparting.

The extension spring (4) and extension shaft (2) are maintained together under pressure, owing to an extension stopper body (3), to the closed end of which an extension spring (4) and an extension shaft (2) atop it are secured, which provides the extension shaft (2) the capability of movement through its open end, which is positioned inside the cascaded upper body (1) and has a inner diameter greater than the outer diameter of the hollow end of said extension shaft (2) and is symmetrical. The extension stopper body (3) is designed in a unidirectional manner so that the extension shaft (2) can run only in a direction away from the closed end of the extension stopper body (3).

The extension spring (4) and extension shaft (2) are maintained together under pressure, owing to a plurality of safety stoppers (3.1), which are prepared on the sides of the extension stopper body (3) facing the movement pins (2.1) and at the same intervals as the movement pins (2.1) and provided in the shape of holes to produce compression on the movement pins (2.1).

The extension shaft (2) is provided to move depending on the force imparted by the body, owing to a cascaded upper body (1), which has a cavity where the extension shaft (2) can move, has one end connected with the bone by means of the fixation pins (6) and has a hollow form.

The extension shaft (2) is enabled to move in a downward direction within certain limits, owing to a plurality of pin steps (1.1), which push the movement pins (2.1) located on the extension shaft (2) inwards, are formed on the inner sides of said cascaded upper body (1) and are placed in the form of projections at reciprocal positions with certain intervals.

The extension stopper body (3) is enabled to push the extension shaft (2) by compressing the extension springs (4) by virtue of the pressure load imparted by the body on the cascaded upper body (1) pushing the pin steps (1.1) and the movement pins (2.1), owing to preferably two stimulus springs (5), which are placed are placed and compressed between a compression step (1.2) formed inside an upper part of the cascaded body (1), and the closed end of the extension stopper body (3) and have the capability of force damping and pressure imparting.

The extension shaft (2) is prevented from moving beyond control in case of an unbalanced load imparted on the cascaded upper body (1) or of the springs (4, 5) being excessively compressed, owing to one load control spring (8), which is located between the open end of the extension stopper body (3) and the cascaded upper body (1) and is capable of shock absorbing.

The body fluid is prevented from leaking through the gap remaining between the cascaded upper body (1) and the extension shaft (2), owing to a protective membrane 7 which is of membrane (7), which is connected between an open end of the cascaded upper body (1) and the extension shaft (2), which is resistant to corrosion and capable of isolation.

In an example application of the mechanical intramedullary fixation pin, a certain load is imparted on the intramedullary pin cascaded body (1) by means of the mechanical stimulus springs (5). By means of the load imparted, the pin steps (1.1) located on the inner wall of the cascaded upper body (1) push the unidirectional movement pins (2.1) located on the extension (distraction) shaft (2) towards the inside. Then the extension (distraction) shaft (2) moves in a direction away from the closed end of the extension stopper body by means of the extension (distraction) springs (4). This movement is gradually controlled by means of the unidirectional movement pin safety stoppers (3.1) located on the extension (distraction) stopper body (3). In this way, the desired amount of extension is provided. Owing to the load control disc springs (8), the amount of the load imparted is controlled in a safe manner. Owing to the protective membrane (7), the body fluid is prevented from entry into the intramedullary pin cascaded body (1). The fixation pins (6) passing through the bone and the intramedullary pin are used for the fixation of the lower and the upper portions.

The invention may not be limited to its representative application provided here. The alternative embodiments may be produced by the persons skilled in the art based on the scope of the claims.

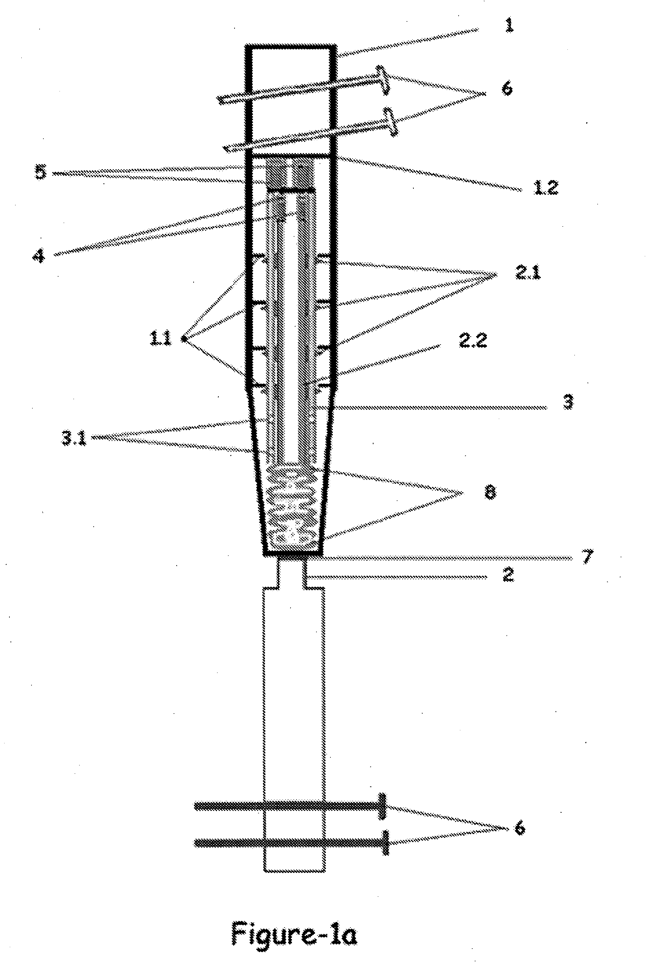

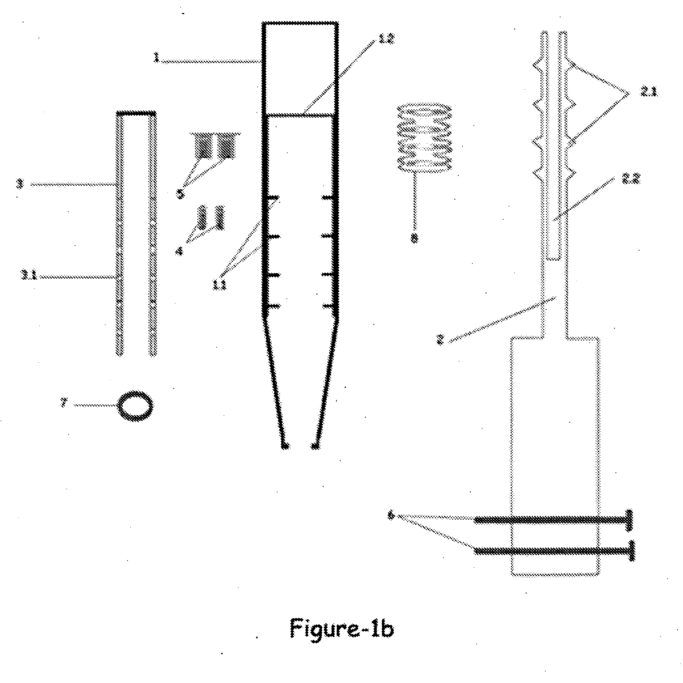

## Claims

1. A bone intramedullary fixation pin used in bone fixations and extensions comprising:
- at least one extension shaft (2);
- at least two fixation pins (6) adapted to attach said extension shaft at one end to a bone;
- at least one hollow end (2.2) at the end portion of said extension shaft (2), which is flexible and can be pushed inwards;
- a plurality of movement pins (2.1), prepared and placed on said hollow end (2.2) in the form of projections at defined intervals; and
- at least one cascaded upper body (1) which has one end adapted to be connected to a bone by means of the fixation pins (6) and has a hollow form, in order to enable said extension shaft (2) to move therein, said cascaded upper body (1) comprising a plurality of pin steps (1.1) which are formed on inner sides of said cascaded upper body (1) and are placed in the form of projections and which are adapted to push the movement pins (2.1) of the hollow end (2.2) of the extension shaft (2) inwards,
**characterized in that** it further comprises:
- at least one extension stopper body (3) having a closed end, an open end and a side wall, said extension stopper body (3) having an inner diameter greater than the outer diameter of the hollow end (2.2) of the extension shaft (2) and comprising a plurality of safety stoppers (3.1) which are provided in the shape of holes in the side wall of said extension stopper body (3) facing the movement pins (2.1) and at the same intervals as the movement pins (2.1), said movement pins (2.1) being engageable into said safety stoppers (3.1) in order to allow said extension shaft (2) to gradually and automatically move through the open end of said extension stopper body (3) in a direction away from the closed end of the extension stopper body (3), within predefined limits.

2. A bone intramedullary fixation pin according to claim 1 **characterized in that** it comprises at least two extension springs (4), adapted to exert pushing force on said extension shaft (2), which are connected to the hollow end (2.2) of said extension shaft (2) and have the capability of force damping and pressure imparting.

3. A bone intramedullary fixation pin according to claim 2
**characterized in that** said extension springs (4) are secured between the closed end of said extension stopper body (3) and the hollow end (2.2) of said extension shaft (2).

4. A bone intramedullary fixation pin according to any one of the preceding claims
**characterized in that** it comprises at least one compression step (1.2) formed inside of an upper part of the cascaded upper body (1).

5. A bone intramedullary fixation pin according to claim 4
**characterized in that** it comprises at least two stimulus springs (5), which are placed and compressed between the compression step (1.2 ) inside the cascaded body (1), and extension stopper body (3) and have the capability of force damping and pressure imparting.

6. A bone intramedullary fixation pin according to any one of the preceding claims
**characterized in that** it comprises at least one load control spring (8), which is located between the open end of said extension stopper body (3) and the cascaded upper body (1) and is capable of shock absorbing.

7. A bone intramedullary fixation pin according to any one of the preceding claims
**characterized in that** it comprises at least one protective membrane (7), which is connected between the open end of said cascaded upper body (1) and the extension shaft (2) said membrane (7) is resistant to corrosion and capable of isolation.

## Patentansprüche

1. Intramedullärer Knochenfixierungsstift, der in Knochenfixierungen und -verlängerungen verwendet wird, umfassend:
- mindestens einen Verlängerungsschaft (2);
- mindestens zwei Fixierungsstifte (6), die dazu ausgebildet sind, den Verlängerungsschaft (2) an einem Ende des Knochens zu befestigen;
- mindestens ein hohles Ende (2.2), das flexibel ist und an dem Endabschnitt des Verlängerungsschaftes (2) nach Innen geschoben werden kann;
- eine Vielzahl von Stiften (2.1), die an dem hohlen Ende (2.2) in der Form von Fortsätzen in definierten Abständen gebildet und angeordnet sind; und
- mindestens einen kaskadenförmigen oberen Körper (1), von dem ein Ende dazu ausgebildet ist, mit Hilfe der Fixierungsstifte (6) mit dem Knochen verbunden zu werden, und eine hohle Form aufweist, so dass sich der Verlängerungsschaft (2) bewegen kann, wobei der kaskadenförmige obere Körper (1) eine Vielzahl von Stiftstufen (1.1) umfasst, die an Innenseiten des kaskadenförmigen oberen Körpers (1) gebildet sind und in der Form von Fortsätzen angeordnet sind und die dazu ausgebildet sind, die Bewegungsstifte (2.1) des hohlen Endes des Verlängerungsschaftes nach Innen zu schieben,
**dadurch gekennzeichnet, dass** er des Weiteren umfasst:
- mindestens einen Verlängerungssperrkörper (3) mit einem geschlossenen Ende und einem offenen Ende und einer Seitenwand, wobei der Körper einen Innendurchmesser aufweist, der größer als der Außendurchmesser des hohlen Endes des Verlängerungsschaftes ist, enthaltend eine Vielzahl von Sicherheitssperren (3.1), die in der Form von Löchern in der Seitenwand des Verlängerungssperrkörpers (3) bereitgestellt sind, die den Bewegungsstiften (2.1) zugewandt sind und dieselben Abstände aufweisen wie die Bewegungsstifte (2.1), wobei die Bewegungsstifte (2.1) mit den Sicherheitssperren (3.1) in Eingriff gebracht werden können, so dass der Verlängerungsschaft (2) sich allmählich und automatisch durch ein offenes Ende des Verlängerungssperrkörpers (3) in eine Richtung weg von dem geschlossenen Ende des Verlängerungssperrkörpers (3) innerhalb vordefinierter Grenzen bewegen kann.

2. Intramedullärer Knochenfixierungsstift nach Anspruch 1, **dadurch gekennzeichnet, dass** er mindestens zwei Verlängerungsfedern (4) umfasst, die eine Schubkraft auf den Verlängerungsschaft (2) ausüben, die mit dem hohlen Ende des Verlängerungsschafts (2) verbunden sind und die Fähigkeit einer Kraftdämpfung und Druckausübung haben.

3. Intramedullärer Knochenfixierungsstift nach Anspruch 2, **dadurch gekennzeichnet, dass** die Verlängerungsfedern (4) zwischen dem geschlossenen Ende des Verlängerungssperrkörpers (3) und dem hohlen Ende des Verlängerungsschaftes (2) befestigt sind.

4. Intramedullärer Knochenfixierungsstift nach einem der vorangehenden Ansprüche, **dadurch gekennzeichnet, dass** er mindestens eine Kompressionsstufe (1.2) umfasst, die an der Innenseite eines oberen Teils des kaskadenförmigen oberen Körpers (1) gebildet ist.

5. Intramedullärer Knochenfixierungsstift nach Anspruch 4, **dadurch gekennzeichnet, dass** er mindestens zwei Stimulusfedern (5) umfasst, die zwischen der Kompressionsstufe (1.2) im Inneren des kaskadenförmigen Körpers (1) und dem Verlängerungssperrkörper (3) angeordnet und zusammengepresst sind und die Fähigkeit einer Kraftdämpfung und Druckausübung haben.

6. Intramedullärer Knochenfixierungsstift nach einem der vorangehenden Ansprüche, **dadurch gekennzeichnet, dass** er mindestens eine Lastkontrollfeder (8) umfasst, die zwischen dem oberen Ende des Verlängerungssperrkörpers (3) und dem kaskadenförmigen oberen Körper (1) angeordnet ist und zum Stoßdämpfung imstande ist.

7. Intramedullärer Knochenfixierungsstift nach einem der vorangehenden Ansprüche, **dadurch gekennzeichnet, dass** er mindestens eine schützende Membran (7) umfasst, die zwischen dem offenen Ende des kaskadenförmigen oberen Körpers (1) und dem Verlängerungsschaft (2) befestigt ist, wobei die Membran (7) korrosionsbeständig und zur Isolierung imstande ist.

## Revendications

1. Broche de fixation intramédullaire osseuse utilisée dans des fixations et extension d'os et comprenant :
- au moins une tige d'extension (2) ;
- au moins deux broches de fixation (6) aptes à rattacher ladite tige d'extension (2) à une extrémité de l'os ;
- au moins une extrémité creuse (2.2) qui est souple et peut être poussée vers l'intérieur de la partie terminale de ladite tige d'extension (2) ;
- une pluralité de broches (2.1) préparées et placées sur ladite extrémité creuse (2.2) sous la forme de projections à des intervalles définis ; et
- au moins un corps supérieur échelonné (1) qui comporte une extrémité apte à être connectée à l'os au moyen des broches de fixation (6) et a une forme creuse afin de permettre à ladite tige d'extension (2) de bouger, ledit corps supérieur échelonné comprenant une pluralité d'échelons de broche (1.1) qui sont formés sur les faces internes dudit corps supérieur échelonné (1) et sont placés sous la forme de saillies et qui sont aptes à pousser vers l'intérieur les broches de mouvement (2.1) de l'extrémité creuse de la tige d'extension,
**caractérisée en ce qu'**elle comprend en outre :
- au moins un corps d'arrêt d'extension (3) ayant une extrémité fermée et une extrémité ouverte et une paroi latérale ; ledit corps ayant un diamètre intérieur supérieur au diamètre extérieur de l'extrémité creuse de la tige d'extension, incluant une pluralité d'arrêts de sécurité (3.1) qui sont prévus sur la forme de creux dans la paroi latérale dudit corps d'arrêt d'extension (3) faisant face aux broches de mouvement (2.1) et avec les mêmes intervalles que les broches de mouvement (2.1), lesdites broches de mouvement (2.1) pouvant s'engager dans lesdits arrêts de sécurité (3.1) afin de permettre à ladite tige d'extension (2) de bouger graduellement et automatiquement dans une extrémité ouverte dudit corps d'arrêt d'extension (3) dans un sens s'éloignant de l'extrémité fermée du corps d'arrêt d'extension (3) dans des limites prédéfinies.

2. Broche de fixation intramédullaire osseuse selon la revendication 1, **caractérisée en ce qu'**elle comporte au moins deux ressorts d'extension (4) qui exercent une force de poussée sur ladite tige d'extension (2), sont connectés à l'extrémité creuse de ladite tige d'extension (2) et ont la capacité d'amortir les forces et d'impartir de la pression.

3. Broche de fixation intramédullaire osseuse selon la revendication 2, **caractérisée en ce que** lesdits ressorts d'extension (4) sont fixés entre l'extrémité formée dudit corps d'arrêt d'extension (3) et l'extrémité creuse de ladite tige d'extension (2).

4. Broche de fixation intramédullaire osseuse selon l'une quelconque des revendications précédentes, **caractérisée en ce qu'**elle comprend au moins un échelon de compression (1.2) formé à l'intérieur de la partie supérieure du corps échelonné supérieur (1).

5. Broche de fixation intramédullaire osseuse selon la revendication 4, **caractérisée en ce qu'**elle comprend au moins deux ressorts d'excitation qui sont placés et comprimés entre l'échelon de compression (1.2) à l'intérieur du corps échelonné (1) et le corps d'arrêt d'extension (3) et ont la capacité d'amortir les forces et d'impartir de la pression.

6. Broche de fixation intramédullaire osseuse selon l'une quelconque des revendications précédentes, **caractérisée en ce qu'**elle comprend au moins un ressort de contrôle de charge (8) qui est localisé entre l'extrémité ouverte dudit corps d'arrêt d'extension (3) et le corps échelonné supérieur (1) et est apte à absorber les chocs.

7. Broche de fixation intramédullaire osseuse selon l'une quelconque des revendications précédentes, **caractérisée en ce qu'**elle comprend au moins une membrane protectrice (7) qui est connectée entre l'extrémité ouverte dudit corps échelonné supérieur (1) et la tige d'extension et que ladite membrane (7) est résistante à la corrosion et apte à l'isolation.
